# EUROPEAN PATENT APPLICATION

(11) **EP 2 410 334 A1**
(43) Date of publication of application: **25.01.2012**
(21) Application number: 11185960.9
(22) Date of filing: 15.02.2008
(51) Int. Cl.: G01N 33/53, G01N 33/567, A61K 49/00, G01N 33/50, G01N 33/574

(54) **T-lymphocyte mitogen for use in a skin test**

(30) Priority: 15.02.2007 US 706946
(62) Divisional of application: 08729957.4
(71) Applicant: IRX Therapeutics, Inc., New York, NY 10019 (US)
(72) Inventor: Hadden, John, New York, NY 11724 (US)
(74) Representative: Austin, Hedley William

(57) **Abstract**

The T lymphocyte is muromonab-CD3 for testing the effect of immunotherapy on a cancer patient in a skin test

## Description

The present invention relates to the field of personalised medicine, and more specifically to a diagnostic skin test for the detection of treatment outcomes.

The present applicants have previously developed a skin test utilizing a natural cytokine mixture (NCM) as described in US Patent 6482389 (hereinafter, the '389 patent). The NCM (also referred to herein as IRX-2), has been previously shown by the present applicants in US Patent 5698194 to be effective in promoting T cell development and function in aged, immunosuppressed mice. Specifically, the NCM was shown to decrease the proportion of immature T cells and increase the proportion of mature T cells in the thymus. The NCM included IL-I, IL-2, IL-6, IL-8, IFN-gamma, and TNF-alpha, as well as GM-CSF, G-CSF, 1 L-3, IL-4, IL-S1 IL-7, and 1 L-12 in trace amounts or absent altogether. The '389 patent discloses a method and kit for determining candidates for immunotherapy, for monitoring the effect of immunotherapy, and for analyzing cell-mediated immunity function in a patient. The method of the '389 patent includes performing two intracutaneous skin tests and reading the skin test after twenty-four hours.

One skin test comprises the administration of a mitogen such as PHA, concanavalin A (ConA), pokeweed antigen (PWA) and other mitogens as known in the art. Response to the PHA skin test reflect the ability of the present T lymphocytes to react to PHA, to release cytokines such as IL-2, and to induce a monocyte and macrophage infiltration leading to the DTH dermal reaction that is observed in the skin test, characteristic of the afferent limb response of the immune system. The second skin test is the administration of NCM and reflects the ability of preformed T cell cytokines to induce the monocyte and macrophage accumulation characteristic of the efferent limb response.

The method of the '389 patent essentially provides a process of monitoring patients with cellular immune deficiency by the steps of determining the result of intracutaneous skin tests with a mitogen such as PHA .and with NCM and the result of blood lymphocyte counts (with or without T-lymphocyte and subset enumeration) to yield a composite "three-dimensional view" of cellular mediated immunity including T lymphocyte number and function (afferent limb) and cytokine production and action on monocytes and macrophages (efferent limb).

Previously, the NCM skin test in conjunction with the PHA skin test was used to predict only a poor response to NCM immunotherapy. In U.S. Patent Application 10/637,869, and European patent 1653912, which claims priority from that US application, the present applicants provided data indicating that cancer patients having a negative intradermal skin test reaction to NCM predicts not only a poor response to immunotherapy but also a poor overall clinical prognosis. However, a certain number of patients were converted from a negative skin test response to a positive one upon treatment with NCM and these converted patients showed improved clinical and pathological responses. It was suggested that a negative skin test to NCM reflects a monocyte defect in the patient, whereby cell-mediated immune responses were deficient, and treatment with NCM can remedy this functional defect.

There still remains a great need for tests, specifically, diagnostics and personalized therapeutics based on reaction to specific antigens, that will reflect the cancer patient's cellular immune status. Thus, one of the objectives of the present invention is to provide a skin test that reflects the efferent limb response, i.e., the monocyte-dependent component of the immune response.

It was previously unknown that other T cell mitogens can be used for predictive skin tests including anti-CD3 monoclonal antibodies. The present invention accordingly provides a T lymphocyte mitogen of muromonab-CD3 for use in a skin test for testing the effect of immunotherapy on a cancer patient. A diagnostic skin test of anti-CD3 monoclonal antibodies is therefore also provided.

The skin test according to the invention may be used in a method of detecting defects in T lymphocyte function, including the steps of administering an effective amount of a T lymphocyte mitogen of muromonab-CD3 to skin, analyzing results of the skin test, and detecting at least one defect in monocyte function, such as a defect in T lymphocyte function.

A mechanism is also provided for indicating either a functioning efferent limb or afferent limb of an immune system including a diagnostic skin test with an effective amount of a T lymphocyte mitogen of muromonab-CD3, respectively.

The skin test according to the invention is useful to determine the appropriate treatment of cancer patients.

According to the invention, muromonab-CD3 is administered as a diagnostic skin test. Preferably, 0.1 to 100 ng of the muromonab-CD3 is administered during the skin test. The diagnostic skin test with muromonab-CD3 is essentially the same as the NCM skin test described above. A positive response to the muromonab-CD3 skin test generally indicates that the afferent limb of the immune system is functioning as it reflects the ability of the present T-lymphocytes to react to muromonab- CD3, to release cytokines such as IL-2, and to induce a monocyte/macrophage infiltration leading to the DTH dermal reaction which is observed in the skin test. Monocytes and macrophages utilize a common final pathway and aid in antigen presentation for the production of antibodies. Thus a positive response indicates that a positive treatment outcome is predicted. A negative response to the muromonab-CD3 skin test generally indicates unresponsiveness to the NCM and immunotherapy and predicts a negative treatment outcome because of T lymphocyte defects. The present invention therefore includes a mechanism for indicating a functioning afferent limb of an immune system including the diagnostic muromonab-CD3 skin test.

There are several specific treatment outcomes that can be predicted through the muromonab-CD3 skin test. For example, the overall survival of the patient can be predicted. A positive response to the muromonab-CD3 skin test favours a major clinical response and that, after treatment with NCM treatment, a patient will survive and remain disease-free. A negative response to the muromonab-CD3 skin test indicates that, even with NCM treatment, a patient's chances of surviving overall are limited.

Also, response to immunotherapy is predicted through the muromonab-CD3 skin test. A positive skin test result indicates that a patient will respond to immunotherapy with NCM, whereas a negative skin test result indicates that the patient will not respond to immunotherapy with NCM.

The muromonab-CD3 skin test also used to predict response to surgery with or without radiotherapy in combination with NCM treatment. A positive skin test result predicts that surgery with or without radiotherapy along with NCM treatment will favour a major clinical response and greater survival in a patient A negative skin test result predicts that surgery with or without radiotherapy along with NCM treatment will not have an impact on a patient's survival.

The muromonab-CD3 skin test further predicts the time to recurrence of disease. A positive skin test result predicts that the time to recurrence will be long, or in other words, recurrence may not occur at all because of the major clinical response predicted. A negative skin test result predicts that the time to recurrence will be short, because the patients will not respond to treatment and their disease will progress. Time to death is also predicted through the use of the muromonab-CD3 skin test. For example, a positive skin test result indicates an elongated period for the time to death of a patient. In other words, the time to death is extended because of the prediction of a positive outcome with NCM treatment. A negative skin test result indicates a shortened period for the time to death of a patient because of predicted non-response to treatment.

A negative muromonab-CD3 skin test can be converted into a positive result by pretreatment with NCM. Thus, administration of NCM after a negative skin test result can correct T lymphocyte defects, and a subsequent positive muromonab-CD3 skin test can show an improvement of the immune system and predict a favourable outcome.

Defects in T lymphocyte function can be detected according to the invention by administering an effective amount of muromonab-CD3 to skin, analyzing results of the skin test, and detecting at least one defect in monocyte function.

The muromonab-CD3 skin test is generally performed by administering an effective amount of muromonab-CD3 to skin, analyzing results of the skin test, and predicting a treatment outcome. The muromonab-CD3 skin test is preferably performed on cancer patients; however, the muromonab-CD3 skin test can also be performed on other immune deficient patients. Administration is generally intradermally, but can be other methods as detailed below. Intradermal injection is preferably into the lower forearm using a 1 cc tuberculin syringe with the needle bevel positioned up. As soon as the bevel is completely covered by intradermal tissue, a small amount of fluid can be injected and the needle advanced slowly administering the remaining volume during advancement.

An effective amount of muromonab-CD3 is preferably 0.1 to 100 ng. The results of the skin test are generally analyzed and read from 6 to 48 hours after administration of the test. Preferably, the test results are analyzed and read 24 hours after administration. Either a negative response or a positive response is obtained. A positive response in general predicts a positive treatment outcome. A negative response in general predicts a negative treatment outcome and at least one defect in T lymphocyte function. From the test results, a specific treatment outcome can be predicted as detailed above, including the overall survival of the patient, response to immunotherapy, response to surgery, response to radiotherapy, time to recurrence, and time to death.

The present invention further enables provision of kits for performing the skin tests described above. A kit for performing the muromonab-CD3 skin test generally includes an effective amount of muromonab-CD3 as described above, preferably 0.1 to 100 ng. The muromonab-CD3 is provided in a pharmaceutically acceptable carrier. The kit also includes the appropriate materials necessary to administer the skin test, such as syringes and needles, as well as control solutions that do not contain the muromonab-CD3 for comparison. The kit can be used to predict any of the treatment outcomes described above of the overall survival of the patient, response to immunotherapy, response to surgery, response to radiotherapy, time to recurrence, and time to death. For any of the above embodiments, the following administration details and/or protocols for treatment are used.

The term "effective amount" for purposes herein is determined by such considerations as are known in the art. The amount must be effective to promote immunization, leading to, e.g., tumour reduction, tumour fragmentation and leukocyte infiltration, delayed recurrence or improved survival rate, or improvement or elimination of symptoms.

As used herein, the term "response" denotes an answer or result to the skin test A response is acquired after analysis of the skin test reaction on the patient. Throughout the application, "response" is used synonymously with "result".

As used herein, the term "skin test" denotes a clinical test performed on a patient which stimulates a response on the patient's skin if a certain set of physiological parameters are present, generally relating to the immune system and a particular disease. The skin tests of the present invention are provided as diagnostic tools and for predicting treatment outcomes.

As used herein, the term "T lymphocyte mitogen" denotes an agent that is capable of stimulating mitosis and lymphocyte transformation. This term is also referred to as a "T cell mitogen". The T lymphocyte mitogen utilized in the present invention is an anti-CD3 monoclonal antibody muromonab-CD3. Muromonab-CD3 (Ortho Biotech), also known under the trade name ORTHOCLONE OKT3(R), is commonly administered to patients receiving organ transplants (such as kidney, heart, or liver transplants) in order to lower the patient's natural immune system. In other words, muromonab-CD3 acts as an immune suppressant. This is necessary to help prevent organ rejection from the body, but it also can make the patient more susceptible to infections. Muromonab-CD3 has not previously been used in the manner of the present invention as a skin test component, as is now detailed in the following illustrative Example.

### EXAMPLE

### Muromonab-CD3 (ORTHOCLONE OKT-3(R)) Skin Test

Three normal human patients were tested with intradermal skin tests of 0.1 ml of anti-CD3 monoclonal antibody ORTHOCLONE OKT-3(R), which is known to be a T cell mitogen in culture at low doses and a T cell suppressant (i.e., an immunosuppressive agent) at high doses in vivo. Skin tests were performed with 1 , 10, and 100 ng of ORTHOCLONE OKT-3(R) and read at 24 hours. Positive reactions to one or more of the doses were observed with approximately 1 cm of erythema and induration. This is the first demonstration that a T cell stimulant other than phytohemagglutin (PHA) can cause a positive skin test and reflect T cell response and efferent limb activation. These data predict that all T cell mitogens will have this reaction and will be useful as a new diagnostic test for the cell mediated immune system.

## Claims

1. A T lymphocyte mitogen of muromonab-CD3 for use in a skin test for testing the effect of immunotherapy on a cancer patient

2. Use according to claim 1, in which the skin test is to be performed with 0.1 to 100 ng of said muromonab-CD3 and optionally a pharmaceutically acceptable carrier.

3. Use according to claim 1 or 2, wherein said skin test is to be administered intradermally.

4. Use according to any of claims 1 to 3, wherein a response to said skin test is to be determined 6 to 48 hours after administration.

5. Use according to any of claims 1 to 4, wherein a response to said skin test indicates at least one defect in monocyte function.
